# EUROPÄISCHE PATENTSCHRIFT

(11) **EP 3 226 682 B1**
(45) Veröffentlichungstag und Bekanntmachung des Hinweises auf die Patenterteilung: **29.09.2021**
(21) Anmeldenummer: 15826128.9
(22) Anmeldetag: 03.12.2015
(51) Int. Cl.: A01K 29/00

(54) **VERFAHREN FÜR DAS GEWINNEN VON INFORMATIONEN ÜBER EIN NUTZTIER**
METHOD FOR OBTAINING INFORMATION ABOUT A FARM ANIMAL
PROCÉDÉ D'ACQUISITION D'INFORMATIONS SUR UN ANIMAL DE RENTE

(30) Priorität: 03.12.2014 AT 8742014; 27.03.2015 AT 1782015
(43) Veröffentlichungstag der Anmeldung: 11.10.2017
(73) Patentinhaber: Smartbow GmbH, 4675 Weibern (AT)
(72) Erfinder: AUER, Wolfgang, 4675 Weibern (AT)
(74) Vertreter: Mannion, Sally Kim
(86) Internationale Anmeldenummer: PCT/AT2015/000155
(87) Internationale Veröffentlichungsnummer: WO 2016/086248

(56) Entgegenhaltungen:
- EP-B1- 1 301 068
- WO-A1-2011/069512
- US-A1- 2010 160 833
- None

## Beschreibung

Die Erfindung betrifft ein Verfahren für das Gewinnen von Informationen über ein Nutztier.

Typischerweise kann das Nutztier an welchem das Verfahren angewandt wird, eine Milchkuh sein; die dabei gewonnenen Informationen betreffen typischerweise die täglich aufgenommene Futtermenge, eine Aussage über Komponenten des aufgenommen Futters, Aussagen bezüglich der Milchleistung, der Zeit der Brunst und des optimalen Besamungszeitpunktes, sowie Aussagen über die Wiederkäutätigkeit.

Für die Durchführung des Verfahrens ist am Kopfbereich eines Tieres ein Beschleunigungssensor angebracht, welcher wiederkehrend Daten misst und wobei die vom Beschleunigungssensor am Tier gemessenen Daten an eine Auswertestation übertragen werden und in der Auswertestation ausgewertet werden.

Durch die US 4618861 A wird schon 1986 der Vorschlag veröffentlicht, ein Tier mit einem Bewegungssensor auszustatten und an Hand der damit automatisch beobachteten Rate von Bewegungen pro Tag darauf zu schließen, ob das Tier - typischerweise ist es eine Kuh - in der Brunst ist oder nicht. Als Bewegungssensor wird beispielsweise ein an einem Halsband umgehängter beschleunigungsabhängiger Schalter vorgeschlagen, welcher immer dann, wenn er um mehr als einen bestimmten Schwellwert beschleunigt wird, einen Zählimpuls liefert. Durch statistische Analyse ist eine Tendenz erkennbar ob Brunst eher schon vorliegt oder eher nicht.

Die EP 2007192 B1 nennt weiterführende Ideen zu dem aus der US 4618861 A bekannten Verfahren. Mittels Sensoren die am Kopf eines Tieres, typischerweise einer Kuh, angeordnet sind, werden eine Mehrzahl von Ausrichtungs- und Bewegungsparametern gemessen (Beschleunigung in verschiedenen Richtungen, Abstand zum Boden etc.) und durch Auswertung dieser Primärdaten darauf geschlossen ob das Tier gehen, steht oder liegt. Charakteristische Änderungen in den Häufigkeiten der Letzteren werden für das Erkennen der Zeiten von Brunst, Fruchtbarkeit und Geburt herangezogen. Optional werden weitere Messungen vorgenommen, beispielsweise über die Dauer von Fressaktivitäten, und damit die Schlüsse erweitert bzw. die Treffergenauigkeit der Schlüsse verbessert.

Durch die DE 36 10 960 A1 wird schon 1987 die Idee veröffentlicht in der landwirtschaftlichen Tierhaltung ein Tier mit einem Sensor auszustatten, welcher eine Zustandsgröße des Tieres wiederholt misst und das Messergebnis an eine Auswertestelle sendet, damit automatisch auf den Gesundheitszustand des Tieres rückgeschlossen und beispielsweise automatisch das Futter angepasst werden kann. Als eine mögliche zu messende Zustandsgröße wird die Beschleunigung genannt; diese wird über die Zeit auf integriert. Das Ergebnis wird als mittlere Eigenbewegung des Tieres interpretiert. Wiederkäuen wird nicht erwähnt.

In der EP 1301068 B1 (Prioritatsjahr 2000) wird vorgeschlagen durch Geräuschanalyse die Dauer des Wiederkäuens eines Tieres innerhalb eines längeren, vorbestimmten Zeitraumes zu erfassen, und daraus Schlüsse auf das Futter oder einen Zustand des Tieres zu ziehen. Wiederkäuen als solches wird durch die Unterschiedlichkeit der Geräusche von Heraufwürgen eines Bolus und Kauen eines Bolus erkannt. Störende Einschränkungen ergeben sich wegen des hohen Energieverbrauchs durch die erforderliche lange Messdauer. Messung von Beschleunigung wird nicht erwähnt.

In der WO2007119070 A1 wird vorgeschlagen eine Mehrzahl von verschiedenen Verhaltensweisen eines Tieres durch Sensoren wie Geräuschsensoren, mehrachsige Beschleunigungssensoren, Temperator sensoren etc. zu erfassen, diese drahtlos an eine Auswerteeinheit zu übertragen und durch Auswertung auf eine Mehrzahl von Zuständen des Tieres rückzuschließen. Es wird vorgeschlagen Wiederkauen an Hand der Ergebnisse von Gerauschsensoren zu erkennen.

In der WO2011069512A1 wird vorgeschlagen, die Aufnahme der Futtermenge eines weidenden Tieres durch Messen einer Kopfbewegung mittels Sensoren, u.a. Beschleunigungssensoren, abzuschätzen. Auf Schluckvorgänge oder Kauschläge im Einzelnen wird dabei nicht eingegangen.

In der EP 2208146 B1 (Priorität 2007) wird vorgeschlagen im äußeren Gehörgang eines Tieres einen mit mehreren Sensoren ausgestatteten Bauteil anzubringen, welcher an zumindest zwei Stellen die Temperatur misst und welcher daneben auch andere Größen wie beispielsweise Geräusche oder Beschleunigungen messen kann. Unter anderem wird vorgeschlagen, durch den Sensor eine Wiederkäuaktivität zu messen; es wird aber nicht weiter erkfärt, wie die Messung realisiert wird und welche Wiederkäuaktivität das genau betrifft.

In der DE 601 33 106 T2 (Deutsche Übersetzung der EP 1 301 068 B1) wird vorgeschlagen, an einem Wiederkäuer mittels Geräuschsensor und Auswertelogik eine die Tätigkeit des Wiederkauens anzeigende Größe zu messen und die Dauer des Wiederkauens innerhalb eines festgelegten Zeitraums automatisch zu erkennen. Die damit gewonnene Erkenntnis wird verwendet um Aussagen zu generieren bezüglich des physiologischen Zustandes des Tieres und/oder bezüglich wünschenswerter Änderungen des Futters um die Milchleistung zu optimieren oder die Gesundheit des Tieres zu erhalten.

In der CH 700 494 B1 (Priorität 2010) wird vorgeschlagen, ein Rind mit einem Halfter auszustatten an dessen Nasenband ein Drucksensor angebracht ist, welcher laufend misst und dessen Messergebnisse durch einen Messlogger aufgezeichnet werden. Auf diese Weise kann Kauaktivität, wie insbesondere Wiederkäuen, erkannt werden, da dabei der gemessene Druckverlauf charakteristisch periodisch schwankt. Nachteilig ist, dass das Halfter sehr fest sitzen muss, damit die Druckmessung verlässlich funktioniert.

Unter der WWW-Adresse http://www.landi.ch/deu/goldmedaille-fuer-quotrumiwatchquot_1250893.shtmi war zumindest am 15.3.2014 eine mit 7.11.2012 datierte Veröffentlichung mit dem Titel "Goldmedaille für Rumiwatch" veröffentlicht. Darin wird das Gerät gemäß der zuvor besprochenen CH 700 494 81 in Kombination mit weiteren Sensoren und mindestens einer Auswerteeinheit beschrieben. Beispielsweise wird überwacht, mit wie vielen Kauschlagen ein Bolus beim Wiederkauen gekaut wird, bevor er wieder geschluckt wird. Eine verringerte Anzahl von Kauschlägen pro Bolus deute auf Verdauungsstörungen oder Fütterungsfehler hin.

In der nach dem Prioritätsdatum der vorliegenden Anmeldung erstveröffentlichten WO 2015041548 A1 wird vorgeschlagen, an einem Tier verschieden Sensoren, unter anderem auch Beschleunigungssensoren anzubringen. Aus der Analyse der Beschleunigungsdaten, unter anderem durch eine Mittelwertbildung über die einzelnen bei einer Fourier-Analyse festgesteliten Frequenzen, wird auf eine Art Wohifühl-Parameter rückgeschlossen. Ebenso wird unter Zuhilfenahme einer Beschleunigungsfrequenz auf das Gewicht des Tieres geschlossen. Es wird auch vorgeschlagen die Kaufrequenz beim Wiederkäuen zu messen; allerdings wird dazu vorgeschlagen Geräuschanalyse anzuwenden.

Die US 2010160833 A1 beschreibt eine Methode für die Analyse des Schluckverhaltens eines Menschen. Ein Beschleunigungssensor ist unmittelbar am Hals einer Person anliegend angebracht und misst jene Vibrationen die dort zufolge von Schluckvorgängen entstehen. Die allgemeine Aufgabenstellung welche zur Erfindung geführt hat, besteht darin, ein Verfahren nach Anspruch 1 für das Gewinnen von Informationen über Kühe bereitzustellen, mit Hilfe derer es automatisch möglich ist, die futteraufnahrne zu überwachen und zu beurteilen, die Milchleistungsfähigkeit einer Kuh ohne direkte Milchmengenmessung zu beurteilen sowie die Brunstzeit und die optimalen Besamungszeitpunkt zu erkennen. Im Vergleich mit Verfahren die es für diese Zwecke schon gibt, soll das neue Verfahren besser verlässliche Informationen liefern und/oder für das Tier und die Betreuungspersonen mit weniger Unannehmlichkeiten und Aufwand verbunden sein.

Eine konkretere, der Erfindung zu Grunde liegende Aufgabe besteht darin, ein Verfahren für das Auswerten von Sensordaten eines in einem Gerät am Kopfbereich eines Wiederkäuers angebrachten Sensors bereitzustellen, wobei der Sensor eine vom Wiederkäuen abhängige Zustandsgröße am Ort der Anbringung des Gerätes misst und die Sensordaten automatisch so aufbereitet werden, dass aus dem Endergebnis relevante Hinweise auf den Gesundheitszustand des Tieres und/oder auf eine Wirkung des durch das Tier aufgenommen Futters erkennbar sind. Gegenüber den dazu vorbekannten Verfahren sollen die Verbesserungen darin bestehen, dass durch das am Tier angebrachte Gerät im zeitlichen Mittel markant weniger elektrische Energie verbraucht wird und dass es keines am Tier mitgeführten Halfters oder ähnlich aufwendigen Haltegerätes bedarf.

Zum Lösen der Aufgabe wird vorgeschlagen, am Kopfbereich des Tieres Beschleunigungsdaten aufzunehmen und diese dahingehend auszuwerten, Schluckvorgänge die das Tier durchführt zu erkennen.

Erstens kann man durch das Zählen der Schluckvorgänge sehr wertvolle Schlüsse ziehen - wie weiter unten näher erläutert. Zweitens wird es durch das Erkennen der Schluckvorgänge möglich, die Zeiten die zwischen Schluckvorgängen liegen zu identifizieren und nur jene Beschleunigungsdaten die von Zeiten zwischen Schluckvorgängen stammen, dahingehend auszuwerten, Informationen über die Kautätigkeit des Tieres zu erhalten. Damit werden die gewonnenen Informationen über Kautätigkeit des Tieres sehr genau und verlässlich und lassen sehr weitreichende Schlüsse zu.

Die Anzahl der Schluckvorgänge pro festgelegtem Zeitraum, typischerweise pro Tag, korreliert sehr direkt mit der in dem Zeitraum aufgenommenen bzw. wiedergekäuten Futtermenge bzw. der getrunkenen Wassermenge. Diese Mengen beeinflussen den Zustand des überwachten Tieres sehr direkt bzw. sind sie durch den jeweiligen Zustand des Tieres sehr direkter beeinflusst. Dadurch haben die aus den Messungen gewonnen Aussagen bezüglich dem Zustand des Tieres eine vergleichsweise sehr hohe Verlässlichkeit.

Die pro Zeitraum aufgenommene Futtermenge korreliert bei Kühen recht direkt mit der zu erwartenden Milchleistung.

Eine verringerte Futteraufnahme pro Zeitraum ist bei gesunden Tieren ein klarer Hinweis auf die Brunst. Indem der Zeitraum der Brunst - nicht nur der Anfang sondern auch das Ende - gut erkennbar ist, ist auch der optimale Zeitraum für die Besamung gut erkennbar. Der optimale Zeitraum für die Besamung fällt mit der Duldungsphase zusammen, welche einen Tag nach dem Ende der Brunsterscheinungen beginnt und bei Kühen, welche an sich eine sehr hohe Futteraufnahme pro Zeitraum haben kürzer ist, beispielsweise vier Stunden, ansonsten etwa zwölf Stunden. Durch das erfindungsgemäß vorgeschlagene Verfahren werden also auch nutzbare Informationen über die Dauer der Duldungsphase gewonnen.

In einer bevorzugten Weiterentwicklung des Verfahrens, werden nicht nur die Schluckvorgänge gezählt, sondern auch die Kauschläge, das sind die bei Kaubewegung durchgeführten Bisse.

Das Verhältnis der Anzahl der Kauschläge zur Anzahl der Schluckvorgänge liefert unter anderem Aussagen über das Verhältnis von Rohfaseranteil zum Eiweißanteil im aufgenommenen Futter und über die Tiergesundheit. Auch die beim Kauen durchgeführten Bisse sind an Hand der Auswertung von Beschleunigungsdaten erkennbar.

In einer bevorzugten Weiterentwicklung des Verfahrens, wird auch dahingehend ausgewertet, ob die festgestellten Schluckvorgänge und ggf. Kauschläge während des Wiederkäuens oder während des Fressens (also der Erstaufnahme von Futter) stattfinden. Auch diese Unterscheidung ist an Hand der Auswertung von Beschleunigungsdaten erkennbar. Die Verhältniszahl der Anzahl der Kauschläge zur Anzahl der Schluckvorgänge ist dann mehr aussagekräftig, bezüglich anteiliger Nahrungskomponenten und/oder Tiergesundheit, wenn sie selektiv es für Fressphasen bzw. Wiederkäuphasen gilt und nicht als Durchschnittswert über beide Phasenarten. Beispielsweise korreliert das nur beim Wiederkäuen gemessene Verhältnis von Kauschlägen zu Schluckvorgängen bekanntermaßen sehr stark mit der Futterqualität, dem pH-Wert im Pansen und auch mit dem Fett- und Eiweißgehalt in der Milch.

In einer bevorzugen Weiterentwicklung des Verfahrens, werden auch Schluckvorgänge erkannt und separat gezählt, die stattfinden weil das Tier trinkt. Auch diese Schluckvorgänge sind an Hand der Auswertung von Beschleunigungsdaten erkennbar. Indem damit Rückschlüsse auf die aufgenommene Wassermenge gezogen werden können, kann der Schluss darauf ob ansonsten eher trockenes raufaserreiches Futter, oder eher saftigeres und damit im Normalfall auch eiweißreicheres Futter aufgenommen wurde, verbessert werden.

Bevorzugt werden Schluckphase und Kauphasen durch eine Varianzanalyse des zeitlichen Verlaufs der gemessenen Beschleunigungsdaten unterschieden. Das Verfahren ist mit Mitteln der automatischen Datenverarbeitung sehr gut durchführbar und liefert verlässlich gute Ergebnisse.

Weiter bevorzugt wird die Anzahl der Kauschläge gemessen indem für den Zeitraum zwischen zwei Schluckvorgängen über den zeitlichen Verlauf der gemessenen Beschleunigungen eine Fourier-Transformation durchgeführt wird und die dabei eruierten Grundfrequenz als die Kauschlagfrequenz gedeutet wird, welche mit dem besagten Zeitraum multipliziert die Anzahl der stattgefundenen Kauschläge ergibt. Das Verfahren ist mit Mitteln der automatischen Datenverarbeitung unter Anwendung der sogenannten FFT (Fast Fourier Transformation) gut durchführbar; es liefert auch bei niedriger Frequenz der zu Grunde liegenden Beschleunigungsmessungen (typischerweise 10 Hz) gut verlässliche Ergebnisse.

Bevorzugt wird der Vorgang des Trinkens durch eine Varianzanalyse der gemessenen Beschleunigungsdaten identifiziert. Dabei wird der zeitliche Verlauf der Varianz berechnet und Schwankungen des Varianzverlaufes in einem bestimmten Frequenzbereich werden als Hinweis auf einen Trinkvorgang gewertet, und die festgestellte Frequenz der Schwankungen wird als Frequenz der Schluckvorgänge beim Trinken interpretiert. Das Verfahren ist mit Mitteln der automatischen Datenverarbeitung gut durchführbar und liefert gut verlässliche Ergebnisse.

In einer bevorzugten Weiterentwicklung des Verfahrens werden an dem Tier nicht nur Beschleunigungen automatisch gemessen, sondern es wird auch überwacht an welchem Ort sich das Tier befindet. In Abhängigkeit davon ob sich das Tier an speziellen Orten befindet und auch eventuell davon wie lange sich das Tier dort schon befindet, werden mehr oder weniger Beschleunigungsdaten aufgenommen und die Auswertung der Beschleunigungsdaten im Hinblick auf das Feststellen bestimmter Handlungen wird intensiviert oder verringert. Beispielsweise brauchen Beschleunigungsdaten nicht im Hinblick auf mögliches Trinken aufgenommen und ausgewertet zu werden, wenn aus der Ortsüberwachung klar hervorgeht, dass sich das Tier an einem Platz befindet, an welchem es sicher nichts zu trinken gibt. Damit lässt sich Energieaufwand für Beschleunigungsmessungen und Datenübertragung verringern sowie Berechnungsaufwand vermeiden, sowie die Vertrauenswürdigkeit von tatsächlich gewonnenen Ergebnissen verbessern.

Das Feststellen an welchem Ort sich das Tier befindet kann beispielsweise mit Hilfe eines an sich bekannten Systems für die Funklokalisierung geschehen, bei welchem das Tier einen Funkknoten mitträgt. Eine einfache und auch hilfreiche Ortsfeststellung kann beispielsweise aber auch mittels am Tier angebrachtem RFID-Transponder und Näherungssensoren für diese Transponder durchgeführt werden. Dabei sind die Näherungssensoren an Plätzen die für interessierende Tätigkeiten kennzeichnet sind, beispielsweise Tränken, Fressplätzen, Liegeplätzen an denen gerne wiedergekäut wird.

In einer besonders bevorzugten Ausführungsweise der Erfindung werden die gewonnenen Beschleunigungsmessdaten dahingehend ausgewertet, den Zahlenwert einer solchen quantifizierbaren Größe zu gewinnen, welche gegebenenfalls einen Momentanzustand des Wiederkäuvorgangs beschreibt. (Unter "quantifizierbare Größe" ist eine Größe zu verstehen zu deren vollständiger Beschreibung eine Zahlenangabe zwingend notwendig ist.)

Die Phase, in welcher ein Wiederkäuer wiederkäut, ist in eine Mehrzahl von zeitlich hintereinander liegenden Einzelzyklen unterteilt, wobei ein Einzelzyklus eine Würgephase, eine Kauphase und eine Schluckphase aufweist. Während der Würgephase wird eine als "Bolus" bezeichnete Menge Futtermittel aus dem Pansen des Tieres in das Maul bewegt. Während der Kauphase wird der Bolus im Maul durch Kaubewegungen feiner zermahlen. Die einzelnen Bisse, die bei den Kaubewegungen durchgeführt werden, werden als "Kauschläge" bezeichnet. Während der Schluckphase wird der Bolus wieder geschluckt. Von außen ist die Kauphase gegenüber den anderen beiden Phasen beispielsweise durch größere, mit der Frequenz der Kauschläge periodische Kopfbewegungen des Tieres unterscheidbar und auch durch charakteristische Geräusche. Da beim Wiederkäuen der Kopf des Tieres charakteristisch bewegt wird, ist das Wiederkäuen durch Beschleunigungsmessung prinzipiell erkennbar.

Die für Beschleunigungsmessung erforderlichen Sensoren sind problemlos mit so geringer Größe zu fertigen, dass sie ohne weiteres in einer Ohrmarke Platz finden (auch einschließlich eines erforderlichen Energiespeichers wie typischerweise einer Batterie).

Indem als Endergebnis der Messung und der Auswertung nicht eine Erkenntnis über die Dauer des Wiederkäuens angestrebt wird, sondern eine quantifizierte Erkenntnis über einen Momentanzustand eines gegebenenfalls gerade stattfindenden Wiederkäuvorganges, reicht es aus, wenn nur in relativ großen zeitlichen Abständen zeitlich relativ kurz gemessen wird. Dadurch kann mit einem vergleichsweise extrem geringen Energieaufwand das Auslangen gefunden werden.

In einer bevorzugten Ausführungsweise ist die durch Auswertung der Sensordaten gewonnene quantifizierbare Größe die Anzahl der Kauschläge pro Bolus. Diese Anzahl korreliert bei Kühen bekanntermaßen mit der Futterqualität, dem pH-Wert im Pansen und auch mit dem Fett- und Eiweißgehalt in der Milch.

In einer anderen bevorzugten Ausführungsweise ist die durch Auswertung der Sensordaten gewonnene quantifizierbare Größe die Frequenz der Kauschläge während der Kauphase in einem Bolus-Zyklus. Diese Frequenz korreliert bei Kühen bekanntermaßen ähnlich wie die Anzahl der Kauschläge pro Bolus mit der Futterqualität, dem pH-Wert im Pansen und auch mit dem Fett- und Eiweißgehalt in der Milch.

Das erfindungsgemäße Verfahren wird im Folgenden an Hand von Zeichnungen und der detaillierten Durchsprache am Beispiel "Wiederkäuen" näher erklärt:
- Fig. 1:: zeigt ein Ablaufdiagramm des zentralen, sich kreisartig wiederholenden Prozessablaufes eines vorteilhaften beispielhaften erfindungsgemäßen Verfahrens.
- Fig. 2:: zeigt einen durch Beschleunigungsmessung an einem gerade wiederkäuenden Tier gemessenen Verlauf des Betrages der Beschleunigung über der Zeit.

Die Phase, in welcher ein Wiederkäuer wiederkäut, ist in eine Mehrzahl von zeitlich hintereinander liegenden Einzelzyklen unterteilt, wobei ein Einzelzyklus eine Würgephase, eine Kauphase und eine Schluckphase aufweist. Während der Würgephase wird eine als "Bolus" bezeichnete Menge Futtermittel aus dem Pansen des Tieres in das Maul bewegt. Während der Kauphase wird der Bolus im Maul durch Kaubewegungen feiner zermahlen. Die einzelnen Bisse, die bei den Kaubewegungen durchgeführt werden, werden als "Kauschläge" bezeichnet. Während der Schluckphase wird der Bolus wieder geschluckt. Von außen ist die Kauphase gegenüber den anderen beiden Phasen beispielsweise durch größere, mit der Frequenz der Kauschläge periodische Kopfbewegungen des Tieres unterscheidbar und auch durch charakteristische Geräusche. Für das erfindungsgemäße Verfahren werden die Kopfbewegungen mit Hilfe von Beschleunigungssensoren gemessen. Die für Beschleunigungsmessung erforderlichen Sensoren sind problemlos mit so geringer Größe zu fertigen, dass sie ohne weiteres in einer Ohrmarke Platz finden (auch einschließlich eines erforderlichen Energiespeichers wie typischerweise einer Batterie).

Die erforderlichen Beschleunigungssensoren stehen im Bedarfsfall natürlich auch für andere Messungen als zu dem hier erfindungsgemäßen Zweck zur Verfügung. Beispiele dazu sind Ganganalysen und die Feststellung sonstiger Tätigkeiten oder Positionen des Tieres wie z.B. fressen, gehen oder liegen.

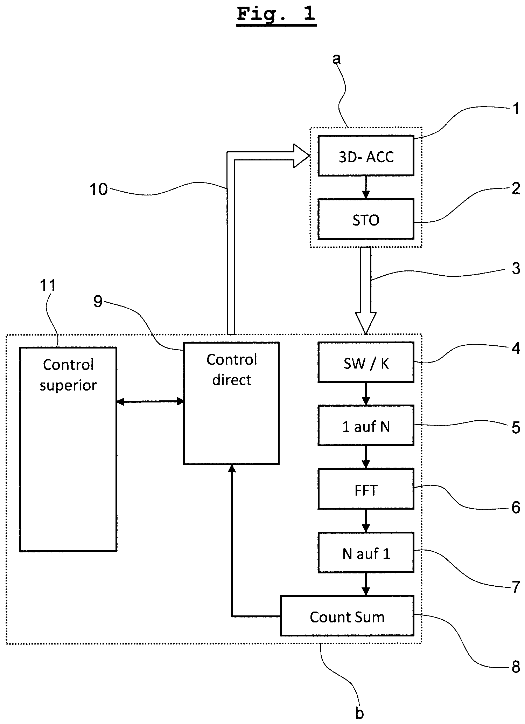

Die Positionsnummern 1 bis 11 gemäß Fig. 1 bedeuten Prozesse, welche in Fig. 1 zusätzlich durch beschriftete Kästchen oder andere Symbole symbolisiert sind und im Wesentlichen in der durch Pfeile gekennzeichneten zeitlichen Reihenfolge hintereinander stattfinden.

Das obere in punktierten Linien dargestellte Rechteck a gemäß Fig. 1 umrandet Symbole 1, 2 für Prozesse, welche jedenfalls in dem am Tier mitgeführten Gerät, stattfinden. Das Rechteck a symbolisiert also ein am wiederkäuenden Tier mitgeführtes Gerät, eine Ohrmarke. Dieses Gerät enthält jedenfalls entweder mehrere eindimensional messende Beschleunigungssensoren oder zumindest einen mehrdimensional messenden Beschleunigungssensor. Das untere in punktierten Linien dargestellte Rechteck b gemäß Fig. 1 umrandet Symbole für Prozesse 4 bis 11, welche idealerweise in einer vom Tier getrennten, typischerweise ortsfesten Auswertestation stattfinden, welche mit dem Gerät am Tier in einer Verbindung 3, 10 steht, über welche bidirektionale Datenübertragung möglich ist. Typischerweise ist diese Verbindung eine drahtlose Funkverbindung. Das Rechteck b symbolisiert also eine Auswertestation.

In Schritt 1 "3D-ACC" werden in drei aufeinander normal stehenden Koordinatenrichtungen Beschleunigungsdaten aufgenommen. Gute Ergebnisse sind erzielbar, wenn die Messfrequenz 10 Hz beträgt, wenn also zehnmal pro Sekunde in jeweils jede der drei Koordinatenrichtungen die jeweilige Beschleunigung gemessen wird.

Schritt 2 "STO" bedeutet das Zwischenspeichern der in Schritt 1 gemessenen Daten in einem Datenspeicher, welcher sich in dem Gerät am Tier befindet. Beispielsweise werden über einen Zeitraum von zwei Minuten Beschleunigungsdaten gemessen und allesamt in den Datenspeicher geschrieben.

In Schritt 3 wird zwischen dem Gerät a am Tier und der typischerweise stationären Auswertestation b eine Funkverbindung aufgebaut und darüber werden die im Gerät a gespeicherten Beschleunigungsdaten an die Auswertestation b übertragen.

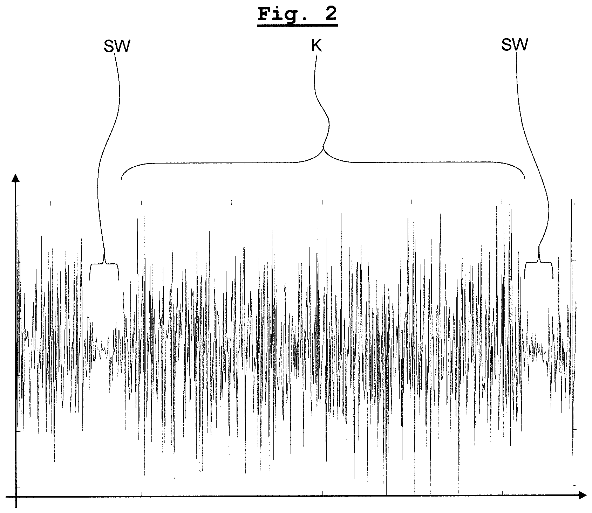

Im Schritt 4 "SW / K" werden die gemessenen Beschleunigungsdaten, welche ja einen zeitlichen Verlauf von Beschleunigungen des Gerätes a repräsentieren, dahingehend ausgewertet, zu erkennen, welche Daten Kauphasen ("K" in Fig. 2) und welche Daten Schluck-Würgephasen ("SW" in Fig. 2) zuordenbar sind. Während der Kauphasen wird ein Nahrungsbolus, welcher sich im Maul des wiederkäuenden Tieres befindet, durch Kaubewegungen fein zermahlen. Während einer Schluck-Würgephase wird erst das fein zerkaute Futtermittel hinuntergeschluckt und dann ein neuer Bolus, welcher anfangs noch aus nicht fein zermahlenem Futtermittel besteht, vom Pansen in das Maul heraufgewürgt. Im Allgemeinen ist die Schluck-Würgephase mit wesentlich kleineren Beschleunigungen und Beschleunigungs-Änderungen verbunden als die Kauphase. Das Unterscheiden zwischen Kauphasen und Schluck-Würgephasen gelingt sehr gut durch eine angepasste Form der Varianzanalyse der Messergebnisse. Dazu wird bevorzugt erst aus den jeweils drei Beschleunigungswerten die je Messzeitpunkt aufgenommen wurden durch geometrische Addition jeweils ein Gesamtbetrag einer Gesamtbeschleunigung errechnet. (Ein sich ergebender Verlauf der absoluten Beschleunigung über die Zeit ist beispielhaft in Fig. 2 gezeigt. In Fig. 2 sind auch zwei Schluck-Würgephasen ("SW") und eine zwischen diesen liegende Kauphase ("K") markiert.) Aus der Gesamtmenge der so gewonnen Datenreihe werden jeweils zwei Teilmengen herangezogen, zu denen die zugehörigen Beschleunigungsmessungen in zwei angrenzenden Zeitfenstern des Messzeitraumes liegen. Von den beiden Teilmengen wird jeweils die Varianz berechnet. (Die Varianz ist die Summe der Quadrate der Abstände der einzelnen Werte zum Mittelwert über alle Einzelwerte, dividiert durch die Anzahl der Einzelwerte). Die beiden gewonnenen Varianzwerte, welche jeweils für eines der beiden Zeitfenster gelten, werden miteinander verglichen. Ein deutlicher Unterschied in den Varianzwerten ist ein Hinweis darauf, dass sich im Bereich der Grenze zwischen den beiden Zeitfenstern ein Übergang zwischen einer Kauphase und einer Schluck-Würgephase befindet. Diese Varianzberechnungen werden für zeitliche verschobene Paare von Zeitfenstern wiederholt, wobei die beiden Fenster eines Paares aber jeweils aneinander angrenzen. Man kann in einer ersten Stufe der Berechnung die Zeitfensterpaare von Varianzberechnung zu Varianzberechnung um eher große Schritte verschieben, sodass der gesamte Zeitbereich schnell durchlaufen wird. Für jene Zeitbereiche für die dabei Hinweise auf Phasenübergänge zwischen Kauphase und Schluck-Würgephase gefunden werden, werden die Varianzberechnungen widerholt, wobei von Berechnung zu Berechnung die Zeitfensterpaare um einen kleineren Zeitsprung verschoben sind, sodass damit der Zeitpunkt des Übergangs zwischen zwei Phasen genauer eingegrenzt werden kann.

Indem die Beschleunigungen ja unabhängig davon gemessen werden, wovon sie ausgelöst wurden, wirken auf das Ergebnis der Beschleunigungsmessungen auch Ereignisse ein, die nichts mit Wiederkäuen zu tun haben, beispielsweise ein Aufschrecken eines Rindes durch ein heftiges Ereignis oder das Schütteln eines Ohres um eine Fliege zu verscheuchen. Damit derartige Ereignisse möglichst nicht auf das Ergebnis der Auswertung der Messungen durchschlagen, werden für die weitere Auswertung des Wiederkäuens nur jene Phasen zwischen zwei als Schluck-Würgephasen interpretierten Phasen als Kauphasen des Wiederkäuens akzeptiert, welche nicht kürzer als eine gewisse Mindestdauer sind (beispielsweise 26 Sekunden) und auch nicht länger als eine gewisse Maximaldauer (beispielsweise 70 Sekunden).

In den Schritten 5 bis 8 wird also zur Messung bezüglich Wiederkauen nur mehr mit Beschleunigungsdaten weiter gearbeitet, welche in Schritt 4 als genau zu einer Kauphase gehörend erkannt wurden. Neben dem Erkennen von Anfang und Ende gehörte dazu auch, dass erkannt wurde, dass es zwischen Anfang und Ende keine Unterbrechung gab und dass Anfang und Ende um eine Zeitdauer auseinander liegen, welche für die Kauphasen beim Wiederkäuen realistisch ist.

Schritt 5 "1 auf N": Zu den einzelnen Messzeitpunkten wurden in Schritt 1 jeweils drei Beschleunigungswerte gemessen, wobei die einzelnen Werte die Beschleunigung in jeweils einer Koordinatenrichtung repräsentieren. Das Wertetrippel einer Messung von einem einzelnen Zeitpunkt bedeutet also einen Vektor im Raum, dessen Richtung von den Größenverhältnissen der einzelnen der drei Beschleunigungsmessungen zueinander abhängt, und dessen Betrag sich aus der geometrischen Addition der Einzelkomponenten ergibt. Von Messzeitpunkt zu Messzeitpunkt ändert sich im Allgemeinen der jeweilige Vektor, welcher durch die drei gemessenen Beschleunigungskomponenten definiert ist, in Richtung und Betrag. In Schritt 5 wird für eine Vielzahl von "igelartig" im Raum angenommenen Richtungen, beispielsweise hundert Richtungen, errechnet, wie groß die in die jeweilige Richtung liegende Komponente der einzelnen gemessenen Beschleunigungsvektoren ist. Dazu kann jeweils das Skalarprodukt des Einheitsvektors (Vektor mit Länge 1) in die jeweilige Richtung mit dem jeweiligen Beschleunigungsvektor errechnet werden.

Über alle Beschleunigungsmessungen einer Kauphase bedeutet das geometrisch, dass aus der einzigen, im dreidimensionalen Raum verlaufenden Kurve, welche gebildet ist, indem die Spitzen der Beschleunigungsvektoren zeitlich geordnet miteinander verbunden wurden, eine Vielzahl von Wegverläufen abgeleitet wird, wobei jeder einzelne Wegverlauf nur genau auf einer Richtungsgeraden im Raum stattfindet und auf jeder Richtungsgerade nur genau ein Wegverlauf stattfindet. (Eine "Richtungsgerade" im Raum in diesem Sinne ist eine Gerade durch den Ursprung des angenommenen Koordinatensystems).

In Schritt 6 "FFT" wird zu jeder der vielen Wegverläufe die in Schritt 5 errechnet wurden und jeweils nur entlang eines Richtungsvektors im Raum stattfinden, eine Fourier-Transformation durchgeführt. D.h. der Verlauf einer skalaren Größe (Betrag der Beschleunigung) über die Zeit wird nicht mehr als Folge von Wertepaaren Zeitpunkt/Betrag der Beschleunigung dargestellt, sondern als Summe von Sinusschwingungen, welche im Einzelnen durch Frequenz, Amplitude und Phase bestimmt sind, wobei in diesem Fall die Amplitude eine Beschleunigung symbolisiert. Für die digitale Datenverarbeitung ist in der Fachwelt als Algorithmus für die Fourier-Transformation vor allem die sogenannte FFT (Fast Fourier Transformation) bestens bekannt und eingeführt, weswegen hier nicht näher darauf eingegangen wird. Das Ergebnis des Schrittes 6 ist also für jeden einzelnen der Beschleunigungsverläufe über die Zeit - die geometrisch jeweils entlang eines einzelnen der Richtungsvektoren ausgerichtet sind - eine eigene Fourier-Darstellung.

Im Schritt 7 "N auf 1" wird aus den einzelnen Fourier-Darstellungen (von denen jede einzelne jeweils für eine Richtung im Raum steht) jene Darstellung herausgefiltert, welche am klarsten eine Frequenzverteilung zeigt, aus welcher der Kaurhythmus des soeben wiederkäuenden Tieres ablesbar ist. Das ist dann der Fall, wenn in jenem Frequenzbereich in welchem die Kaufrequenz erfahrungsgemäß liegt, entweder eine einzige Schwingung bezüglich der Amplitude über alle anderen Schwingungen in diesem Frequenzbereich klar dominiert, oder wenn in diesem Frequenzbereich nur sehr wenige, bzgl. ihrer Frequenz lokal zusammenliegende Schwingungen, bezüglich der Amplitude über alle anderen Schwingungen in diesem Frequenzbereich klar dominieren. Im ersten Fall ist die Frequenz der einzelnen Schwingung die gesuchte Kaufrequenz. Im zweiten Fall kann die Kaufrequenz bestimmt werden indem in der Fourier-Darstellung durch jene Punkte durch welche die drei dominantesten Schwingungen definiert sind (Abszisse entspricht Frequenz, Ordinate entspricht Amplitude) eine Parabel gezogen wird, deren Symmetrieachse zur Ordinate parallel liegt. Der Abszissenwert des Ortes des Scheitelpunktes der Parabel ist die gesuchte Kaufrequenz.

Bei Rindern liegt die Kaufrequenz beim Wiederkäuen im Bereich von etwa 40 bis etwa 85 Kauschlägen pro Minute (0,67 bis 1,42 Hz), typischerweise im Bereich von 60 bis 70 Kauschlägen pro Minute (1 bis 1,17 Hz).

In Schritt 8 "Count Sum", können Zähler nachgestellt und gewonnene Ergebnisse ausgegeben werden. Die Ausgabe eines Ergebnisses bezüglich des momentanen Wertes einer das Wiederkäuen beschreibenden Größe kann die Kaufrequenz sein oder besser noch die Anzahl der Kauschläge pro Bolus. Um die Anzahl der Kauschläge pro Bolus angeben zu können, ist die Kaufrequenz mit der in Schritt 4 festgestellten Dauer der Kauphase zu multiplizieren.

Ein Zähler zählt die beim Wiederkäuen stattfindenden Kauschläge. Zu dem dort schon in diesem Zähler befindlichen Wert wird die Anzahl der im letzten Verarbeitungszyklus festgestellten Kauschläge hinzugezählt. Diese Anzahl ergibt sich als die errechnete Kaufrequenz multipliziert mit der Dauer der betreffenden Kauphase, welche durch zwei Schluck-Würgephasen begrenzt wird, - deren Erkennung in Punkt 4 stattfand.

Ein zweiter Zähler zählt die beim Wiederkäuen stattfindenden Schluckvorgänge. Zu dem dort schon in diesem Zähler befindlichen Wert wird je Schluck-Würgephase der Wert 1 hinzugezählt.

In Schritt 9 "Control direct" wird durch eine erste, untergeordnete Steuereinheit festgelegt, wann durch das am Tier mitgeführte Gerät a wieder ein Messversuch gestartet werden soll und eventuell auch wie lange gegebenenfalls gemessen werden soll. Die Regeln nach denen das in Schritt 9 geschieht, werden gemäß dem skizzierten Beispiel durch einen übergeordneten logischen Prozess 11 "Control superior" festgelegt.

Für die Überwachung des Zustandes eines Tieres mittels Bestimmung der Anzahl der Bisse je Bolus beim Wiederkäuen, ist es nicht erforderlich dauerhaft zu messen. Es kann beispielsweise reichen, wenn drei oder viermal am Tag die Anzahl der Kauschläge je Bolus beim Wiederkäuen erfolgreich festgestellt wird.

Sofern es um das Herausfinden des Momentanwertes einer das Wiederkäuen beschreibenden Größe geht, ist idealerweise eine Logik hinterlegt, welche so lange am Tag häufiger Messvorgänge startet (beispielsweise jede halbe Stunde) bis die Anzahl der gelungenen Messvorgänge, bei denen tatsächlich Wiederkäuen festgestellt und analysiert wurde, das für einen Tag erforderliche Ausmaß erreicht hat. Natürlich kann diese Logik auch von weiteren Vorgängen beeinflusst sein wie beispielsweise Alter des Tieres, Hinweise auf Brünstigkeit oder zeitliche Nähe zu einem Geburtsvorgang oder Verdacht auf Krankheiten. Idealerweise gibt es auch die Möglichkeit diese Logik über ein Eingabegerät durch Menschen zu editieren. Das beschriebene Verfahren ist bezüglich Energieverbrauch in dem am Tier befindlichen Gerät a deswegen hoch effizient, weil die Messungen nicht dauerhaft durchgeführt werden müssen, sondern nur ein paarmal pro Tag, sodass die Betriebszeit des Gerätes a pro Tag beispielsweise im Bereich von 1% der Tagesdauer liegen kann. Damit ist erreichbar, dass die im Gerät a befindliche Batterie sehr, sehr lange halten kann.

In Schritt 10 wird eine Funkverbindung zwischen dem (stationären) Gerät b und dem am Tier befindlichen Gerät a aufgebaut und dem Gerät a werden die das Gerät a betreffenden Festlegungen mitgeteilt. Sofern nicht ohnedies kontinuierlich gemessen wird betrifft dies zumindest den Zeitpunkt des nächsten Messzyklus.

Die beschriebene Auswertung der Messdaten gemäß den Schritten 4 bis 8 erscheint als aufwändig. Tatsächlich ist sie aber leicht durchzuführen, da sie an einem stationären Gerät, beispielsweise einem PC durchgeführt werden kann und da in diesem spielend leicht ausreichend Rechenkapazität, Energie und Zeit bereitgestellt werden kann. Durch die hoch entwickelte Auswertung ist die Gefahr von Fehlmessungen sehr gering und die erreichte Messgenauigkeit ist sehr gut, obwohl man am Tier mit wenigen, relativ kurzen Messzyklen, bei recht niedriger Messfrequenz (typischerweise 10 Hz) das Auslangen findet.

Das beschriebene Verfahren für das Herausfinden des Momentanwertes einer das Wiederkäuen beschreibenden quantifizierbaren Größe ist innerhalb des Erfindungsgedankens in mancherlei Hinsicht abwandelbar und/oder ausbaubar. Es sei dazu genannt:
Beispielsweise könnte man bei der Auswertung der Daten auf die Umwege über die Projektion der Beschleunigungsvektoren auf viele einzelne Richtungsvektoren (Schritt 5), die Fourier-Transformationen der Beschleunigungen je einzelnem Richtungsvektor und die Suche der am besten aussagekräftigen Fourier-Transformation verzichten, und stattdessen mit einer höheren Messfrequenz in Gerät a Beschleunigungen messen (etwa 100 Messungen pro Sekunde anstatt 10 Messungen) und durch Größenvergleich aufeinanderfolgender Messergebnisse die Zeitpunkte von Extremwerten herausfinden und aus deren Abständen auf die Frequenz von Schwingungen und letztendlich auf die Kaufrequenz oder die Dauer von einzelnen Kauschlägen schließen. Allerdings würde man wegen der höheren Messfrequenz mehr Energie in Gerät a verbrauchen und wegen der geringeren Verlässlichkeit der gewonnenen Messergebnisse müsste man sehr viel öfter Messungen durchführen und versuchen, durch Durchschnittsbildung und weitere logische Filter, ausreichende Messgenauigkeit und Verlässlichkeit der Richtigkeit zu erreichen. Für das Erreichen gleich guter Genauigkeit und Verlässlichkeit hätte man damit drastisch mehr Energieverbrauch pro Tag in dem am Tier befindlichen Gerät a; vermutlich müsste man auch die Hardware im Gerät a aufwändiger ausführen, sodass mehr Beschleunigungsmessungen pro Zeit durchgeführt werden können und dass mehr Messergebnisse zwischengespeichert werden können. Vermutlich fände man aber dennoch mit einem geringeren Energieaufwand das Auslangen, als wenn man einfach allgemein die Dauer des Wiederkäuens pro Tag messen würde.

Beispielsweise kann am Gerät a vor einem durchzuführenden Messzyklus zur Aufnahme der Beschleunigungen während des Wiederkäuens von zumindest einem Bolus, ein wesentlich kürzerer Messzyklus gefahren werden, durch welchen festgestellt wird, ob überhaupt ausreichend Beschleunigungen vorliegen, dass Wiederkäuen vorliegen kann. Die diesbezügliche Auswertung, welche beispielsweise darin bestehen kann, dass Messwerte der Beschleunigungen über eine kurze Dauer aufsummiert werden und das Ergebnis mit einem Schwellwert verglichen wird, kann auch schon an dem am Tier befindlichen Gerät a durchgeführt werden. Wenn dabei festgestellt wird, dass gerade sicher nicht wiedergekäut wird, kann die anstehende Messung bezüglich Wiederkäuens zeitlich um eine festgelegte Dauer, beispielsweise eine halbe Stunde, nach hinten verschoben werden.

Die Datenübertragung zwischen dem am Tier befindlichen Gerät a und der Auswertestation b braucht nicht unbedingt drahtlos über Funk zu erfolgen. Sie kann beispielsweise auch durch elektrische Leitung unter Miteinbeziehung des Körpers des Tieres als elektrischen Leiter erfolgen, sobald das Tier eine Elektrode, die mit der Auswertestation in Kontakt ist, berührt oder ihr zumindest so nahe kommt, dass kapazitive Signalübertragung möglich ist.

Es ist auch möglich Fourier-Transformation an den Rohdaten der Beschleunigungsmessungen durchzuführen oder an geringer als gemäß Fig. 1 weiterverarbeiteten Daten aus den Beschleunigungsmessungen. Auch aus den diesbezüglichen Ergebnissen ist die Frequenz der Kauschläge gegebenenfalls einigermaßen herauslesbar, allerdings ist das Ergebnis weniger genau und weniger eindeutig als gemäß dem an Hand Fig. 1 beschriebenen Ablauf.

Für die durch Auswertung der Sensordaten gewonnene quantifizierbare Größe, welche gegebenenfalls etwas über einen Momentanzustand des Wiederkäuvorganges aussagt, kommen innerhalb des Erfindungsgedankens neben den schon beschriebenen Größen "Anzahl der Kauschläge pro Bolus" und "Kaufrequenz" beispielsweise auch die folgende Größen in Frage: Länge der Kauphase, Länge der Würgephase, Länge der Schluckphase, Verhältnis besagter Phasenlängen zueinander, gemittelte Größen von Beschleunigungen während einzelner Phasen, Verhältnisse von Größen von Beschleunigungen bei unterschiedlichen Phasen.

Das im Detail für das Messen des Wiederkäuens beschriebene Verfahren ist weitgehend analog anwendbar für das Messen der Futteraufnahme, also des Fressens. Auch die Futteraufnahme läuft in sich wiederholenden Folgen von jeweils mehreren Kauschlägen und einem Schluckvorgang ab. Unterschiede zum Wiederkäuen betreffen die Frequenz der Kauschläge (diese ist höher als beim Wiederkäuen), sowie die Dauer zwischen zwei Schluckvorgängen (diese ist kürzer als beim Wiederkäuen), sowie die Tatsache, dass ein Schluckvorgang bei der Futteraufnahme weniger Zeit beansprucht als ein Schluck-Würgevorgang beim Wiederkäuen.

Bei der Futteraufnahme ist eine niedrigere Anzahl von Kauschlägen zwischen zwei Schluckvorgängen ein Hinweis auf eiweißreicheres Futter; eine größere Anzahl von Kauschlägen zwischen zwei Schluckvorgängen hingegen ist ein Hinweis auf Futter mit höherem Langfaseranteil. Anstatt bei der Futteraufnahme die Kauschläge zu zählen, kann man auch die Dauer zwischen aufeinanderfolgenden Schluckvorgängen messen. Die Aussagen dieser Dauer über das Futter und den Zustand des Tieres sind tendenziell mit den Aussagen der Verhältniszahl aus Kauschlägen und Schluckvorgängen vergleichbar, weil eine längere Dauer zwischen zwei Schluckvorgängen mit einer höheren Anzahl von Kauschlägen zwischen zwei Schluckvorgängen einhergeht. Als Basis für einen Vergleich zwischen verschiedenen Tieren scheint jedoch nach bisherigen Beobachtungen die Zahl der Kauschläge pro Schluckvorgang besser aussagekräftig zu sein als die Dauer zwischen zwei Schluckvorgängen. Scheinbar schwankt die die Kaufrequenz von Tier zu Tier stärker als die bei einem Schluckvorgang bewegte Futtermenge.

Das Trinken ist aus den gemessenen Beschleunigungsdaten recht gut durch eine Varianzanalyse in Form des Vergleichs der Varianz innerhalb von zwei aneinander angrenzenden Zeitfenstern - wie weiter oben am Beispiel Wiederkäuen beschrieben - erkennbar. Bei relativ niedrigen Beschleunigungswerten gibt es einen sich in einem langsamen Rhythmus wiederholenden Ablauf aus einer längeren Saugphase bei der nahezu keine Beschleunigung stattfindet und einer kürzeren Schluckphase mit höherer Beschleunigung. Die Periodendauer eines aus Saugen und Schlucken bestehenden Zyklus' beträgt bei Kühen typischerweise 5 bis 10 Sekunden.

Der übergeordnete logische Prozess 11 "Control superior" ist ein übergeordneter Steuerungsprozess. Beispielsweise können in diesem weitere Einflussgrößen als nur Zeit und Beschleunigungswerte berücksichtigt werden. Typischerweise werden Ergebnisse einer Lokalisierung berücksichtigt und logisch verarbeitet, sowie auch Vorgaben die editierbar an einem Userinterface festgelegt werden. Durch den Prozess 11 können parallel oder alternativ zu den Prozessschritten 4 bis 8 weitere Prozessschritte gesteuert werden, durch welche die gemessenen Beschleunigungsdaten dahingehend untersucht und ausgewertet werden ob und wenn ja wie Futteraufnahme oder Trinken stattfindet.

Ebenso kann durch den übergeordneten Prozess 11 erkannt werden - typischerweise an Hand von Beschleunigungsdaten, ob überhaupt Wiederkäuen, Futteraufnahme oder Trinken vorliegt. Wenn längere Zeit nichts davon vorliegt, kann das Aufnehmen und detaillierte Analysieren von Beschleunigungsmessdaten eingeschränkt werden, also nur in größer beabstandeten Zeiträumen "probeweise-" durchgeführt werden. So kann insbesondere in Schlafphasen Energie im Gerät a gespart werden. Das Gerät a ist ja mobil am Tier mitgeführt und deswegen auf eine Batterie oder einen Akkumulator angewiesen.

Im Prozess 11 werden auch die Zählergebnisse von Schritt 8 gespeichert und ggf. zu weiteren Aussagen, erforderlichenfalls Alarmen etc. weiter verarbeitet. Ebenso werden gegebenenfalls Ergebnisse bezüglich Futteraufnahme oder Trinken gespeichert und weiter verarbeitet.

Durch das Weiterverarbeiten werden beispielsweise folgende Aussagen generiert, welche auch ausgegeben werden können:
Anzahl der Schluckvorgänge pro Tag
Anzahl der Schluckvorgänge bei der Futteraufnahme pro Tag
Anzahlverhältnis Kauschläge pro Schluckvorgang beim Wiederkäuen
Anzahlverhältnis Kauschläge pro Schluckvorgang bei der Futteraufnahme
Anzahl der Schluckvorgänge beim Trinken
Abweichungen vom Durchschnittswert der einzelnen Anzahlen bzw. Anzahlverhältnisse gegenüber einem Normwert (bzw. dem jeweiligen Herdendurchschnittswert) Abweichungen vom Durchschnittswert der einzelnen Anzahlen bzw. Anzahlverhältnisse gegenüber dem Durchschnittswert beim selben Tier in vergangenen (wählbaren) Zeiträumen.
Aussagen worauf die obigen Abweichungen wie stark ein Hinweis sind beispielsweise Brunst ja/nein, prognostizierter optimaler Besamungszeitraum, zu viel oder zu wenig Eiweiß, .....
Sonstige Aussagen zum Zustand: gesunder Normalzustand, mutmaßliche Milchleistung,...

Bisher wurde das erfindungsgemäße Verfahren vor allem an Hand der Anwendung für Milchkühe erklärt und beschrieben.

Im Rahmen des fachmännischen Handelns ist es auch für die Anwendung an anderen Tieren adaptierbar. Im Wesentlichen sind für dieses Adaptieren die kennzeichnenden Beschleunigungsbeträge, Varianzen und Wiederholfrequenzen von Vorgängen zu identifizieren und die Auswerteverfahren dementsprechend zu justieren.

## Patentansprüche

1. Verfahren für das Gewinnen von Informationen über ein Nutztier, wobei am Kopfbereich eines Tieres ein Gerät (a) angebracht ist, weiches mindestens einen Beschleunigungssensor beinhaltet, durch welchen wiederkehrend Beschleunigungsdaten gemessen werden, wobei die Beschleunigungsdaten mit Mitteln der automatisierten Datenverarbeitung ausgewertet werden und als Ergebnis der Auswertung auf Tätigkeiten und/oder Zustände des Tieres geschlossen wird,
**dadurch gekennzeichnet, dass**
der Beschleunigungssensor in einer Ohrmarke angeordnet ist und dass die aufgenommen Beschleunigungsdaten dahingehend ausgewertet werden, Schfuckvorgänge die das Tier durchführt zu erkennen.

2. Verfahren nach Anspruche 1, **dadurch gekennzeichnet, dass** Beschleunigungsdaten dahingehend ausgewertet werden, dass Zeiträume in welchen das Tier schluckt von Zeiträumen unterschieden werden an denen das Tier kaut und dass dazu die Varianzen der gemessenen Beschleunigungsdaten innerhalb von aneinander angrenzenden Zeitfenstern berechnet und verglichen werden.

3. Verfahren nach Anspruch 1 oder 2, **dadurch gekennzeichnet, dass** die Schluckvorgänge gezählt werden.

4. Verfahren nach einem der Ansprüche 1 bis 3, **dadurch gekennzeichnet, dass** die aufgenommen Beschleunigungsdaten zusätzlich dahingehend ausgewertet werden, Kauschlage die das Tier durchführt zu erkennen und zu zählen.

5. Verfahren nach einem der Ansprüche 2 bis 4, **dadurch gekennzeichnet, dass** Zählwerte generiert werden, die sich jeweils auf einen vorherbestimmten Zeitraum beziehen, insbesondere auf Zeiträume mit jeweils 24 Stunden Dauer.

6. Verfahren nach einem Anspruch 4 oder 5, **dadurch gekennzeichnet, dass** die in einem Zeitraum stattfindende Anzahl von Kauschlägen errechnet wird indem für diesen Zeitraum über den zeitlichen Verlauf der gemessenen Beschleunigungen eine Fourier-Transformation durchgeführt wird, die dabei eruierte Grundfrequenz als Kauschlagfrequenz gedeutet wird und mit der Länge des Zeitraums multipliziert wird.

7. Verfahren nach Anspruch 6, **dadurch gekennzeichnet, dass** Beschleunigung in jeweils drei Koordinatenrichtungen gemessen wird und dass für eine Mehrzahl von im Raum angenommenen, zueinander unterschiedlichen Richtungen und für die zu den einzelnen Messzeitpunkten gemessenen Beschleunigungsvektoren der Betrag jener Richtungskomponente des jewel-ligen Beschleunigungsvektors errechnet wird, welche zu der jeweiligen im Raum angenommenen Richtung parallel liegt, sodass zu jeder der im Raum angenommenen Richtun-gen jeweils eine Datenreihe über die zeitliche Abfolge der in die jeweilige Richtung ausge-richteten Beschleunigungskomponenten gebildet wird, und dass die Fourier-Transformation an einer oder mehrerer dieser Datenreihen durchgeführt wird.

8. Verfahren nach Anspruch 5 und Anspruch 6 oder Anspruch 7, **dadurch gekennzeichnet, dass** der Zeitraum über welchen Kauschläge gezählt werden, vom Ende einer Schluckphase bis zum Beginn der nächsten Schluckphase reicht.

9. Verfahren nach einem der Ansprüche 1 bis 8, **dadurch gekennzeichnet, dass** Schluckvorgänge die Teil eines Trinkvorganges sind, separat gezählt werden.

10. Verfahren nach Anspruch 9, **dadurch gekennzeichnet, dass** der Vorgang des Trinkens durch Varianzanalyse der gemessenen Beschleunigungsdaten identifiziert wird, wobei Schwankungen des Varianzverlaufes innerhalb eines vorbestimmten Frequenzbereiches als Hinweis auf einen Trinkvorgang gewertet werden.

11. Verfahren nach einem der Ansprüche 1 bis 10, **dadurch gekennzeichnet, dass** es an einem Wiederkäuer angewendet wird und dass die Beschleunigungsdaten auch dahingehend ausgewertet werden, zu erkennen ob die Schluckvorgange und gegebenenfalls Kauschläge während der Futteraufnahme oder während des Wiederkäuens stattfinden und dass Schluckvorgänge im Rahmen der Futteraufnahme separat von Schluckvorgängen im Rahmen des Wiederkäuens gezählt werden.

12. Verfahren nach Anspruch 3 und einem der weiteren Ansprüche 1 bis 11, **dadurch gekennzeichnet, dass** in einem automatisierten Datenverarbeitungsprozess an Hand der gemessen Zählwerte und hinterlegter weiterer Daten eine oder mehrere der folgenden Aussagen bezüglich des Tieres generiert werden:
- Anzahl der Schluckvorgänge pro Tag
- Anzahl der Schluckvorgänge bei der Futteraufnahme pro Tag
- Anzahlverhältnis Kauschläge pro Schluckvorgang beim Wiederkäuen
- Anzahlverhältnis Kauschläge pro Schluckvorgang bei der Futteraufnahme
- Anzahl der Schluckvorgänge beim Trinken
- Abweichungen vom Durchschnittswert der einzelnen zuvor genannten Anzahlen bzw. Anzahlverhältnisse gegenüber einem Normwert und/oder einem Herdendurchschnittswert
- Abweichungen vom Durchschnittswert der zuvor genannten Anzahlen bzw. Anzahlverhältnisse gegenüber dem Durchschnittswert beim selben Tier In vergangenen Zeiträumen.
- Aussagen über einen Zustand des Tieres für welchen die zuvor genannten Anzahlen und/oder Anzahlverhältnisse und/oder Abweichungen ein Hinweis sind, insbesondere Aussagen auf die Brunstzeit, Aussagen auf den prognostizierten optimalen Besamungszeitraum, Aussagen über das Verhältnis von Langfasergehalt zu Eiweißgehalt im Futter, Aussagen über die mutmaßliche Milchleistung, Aussagen über den Gesundheitszustand.

13. Verfahren nach einem der Ansprüche 1 bis 12, **dadurch gekennzeichnet, dass** das Nutztier ein Wiederkäuer ist, und dass die aufgenommen Beschleunigungsdaten dahingehend ausgewertet werden, die Maßzahl einer quantifizierbaren Größe festzustellen, welche einen Momentanzustand eines Wiederkäuvorganges beschreibt.

14. Verfahren nach Anspruch 13, **dadurch gekennzeichnet, dass** die durch Auswertung gewonnenen quantifizierten Daten die Frequenz der Kauschläge während des Wiederkauens betreffen.

15. Verfahren nach Anspruch 13 oder 14, **dadurch gekennzeichnet, dass** durch Auswertung der Beschleunigungsdaten darauf geschlossen wird, ob die einzelnen Beschleunigungsdaten einer Kauphase (K) oder einer Schluck-Würgephase (SW) eines Wiederkäuvorganges zuzuordnen sind und dass weitere Auswertungen nur mit jenen Messdaten durchgeführt werden, welche einer Kauphase (K) zugeordnet wurden.

16. Verfahren nach Anspruch 15, **dadurch gekennzeichnet, dass** über zwei Zeitfenster, weiche aneinander angrenzen und jeweils eine Teildauer der Messdauer von Beschleuhigungsdaten betreffen, die Varianz der Messdaten oder eine mit der Varianz der Messdaten korrelierende Größe berechnet wird und dass starke Unterschiedlichkeit der beiden damit berechneten Größenwerte als Wechsel zwischen einer Kauphase (K) und einer Schluck-Würgephase (SW) während des Wiederkauens interpretiert wird.

17. Verfahren nach Anspruch 15 oder 16, **dadurch gekennzeichnet, dass** die Auswertung zwecks Unterscheidung der Zuordnung der Messdaten zu einer Kauphase (K) oder einer Schluck-Würgephase (SW) anhand der Reihe jener zeitlich aufeinanderfolgenden Beschleunigungswerte verwendet wird, welche jeweils den Betrag der Gesamtbeschleunigung zu einem Messzeitpunkt bedeuten und für den jeweiligen Messzeitpunkt durch geometrische Addition der zum jeweiligen Messzeitpunkt: in den einzelnen Koordinatenrichtungen gemessenen einzelnen Beschleunigungswerte berechenbar sind.

18. Verfahren nach Anspruch 7 und einem der Ansprüche 13 bis 17, **dadurch gekennzeichnet, dass** für eine Vielzahl von Datenreihen, weiche jeweils einer anderen Richtung im Raum zugeordnet sind, jeweils eine Fourier-Transformation durchgeführt wird und dass aus der gewonnenen Vielzahl von Ergebnissen jenes herausgefiltert wird, bei welchem in jenem Frequenzbereich in welchem die Frequenz der Kauschläge prinzipiell liegen kann, eine einzige Schwingung bezüglich Amplitude über alle anderen Schwingungen in diesem Frequenzbereich am stärksten dominiert und dass die Frequenz dieser dominierenden Schwingung als die Frequenz der Kauschläge interpretiert wird.

19. Verfahren nach Anspruch 7 und einem der Ansprüche 13 bis 17, **dadurch gekennzeichnet, dass** für eine Vielzahl von Datenreihen, weiche jeweils einer anderen Richtung im Raum zugeordnet sind, jeweils eine Fourier-Transformation durchgeführt wird und dass aus der gewonnenen Vielzahl von Ergebnissen jenes herausgefiltert wird, bei welchem in jenem Frequenzbereich in welchem die Frequenz der Kauschläge prinzipiell liegen kann, drei Schwingungen die bezüglich Frequenz nahe beieinander liegen, bezüglich Amplitude über alle anderen Schwingungen in diesem Frequenzbereich am stärksten dominieren und dass als Frequenz der Kauschläge jene Frequenz Interpretiert wird, an welcher in der Fourier-Darstellung der Scheitelpunkt jener Parabel mit zur Ordinate parallelen Symmetrieachse liegt, welche durch jene Punkte verläuft, welche die besagten drei Schwingungen bezüglich Frequenz und Amplitude kennzeichnen.

20. Verfahren nach Anspruch 15 und Anspruch 18 oder 19, **dadurch gekennzeichnet, dass** die Anzahl der Kauschläge pro Futtermittelportion die aus dem Pansen in das Maul hochgewürgt wird, gekaut wird, dann wieder geschluckt wird, errechnet wird, indem die Frequenz der Kauschläge mit der Dauer der Kauphase multipliziert wird.

## Claims

1. A method for obtaining information about a farm animal, wherein a device (a) is attached to the head area of an animal, which device contains at least one acceleration sensor by means of which acceleration data is measured repeatedly, wherein the acceleration data is evaluated with automated data processing means and activities and/or states of the animal are inferred as a result of the evaluation,
**characterized in that**
the acceleration sensor is arranged in an ear tag and the recorded acceleration data is evaluated to the effect that swallowing processes which the animal carries out are detected.

2. The method according to claim 1, **characterized in that** acceleration data is evaluated to the effect that time periods in which the animal swallows are differentiated from time periods in which the animal chews, and **in that** for this purpose the variance between the measured acceleration data within adjoining time windows is calculated and compared.

3. The method according to claim 1 or 2, **characterized in that** the swallowing processes are counted.

4. The method according to any one of claims 1 to 3, **characterized in that** the recorded acceleration data is additionally evaluated to the effect that chewing actions which the animal carries out are detected and counted.

5. The method according to any one of claims 2 to 4, **characterized in that** counting values which each relate to a previously determined time period, in particular to time periods which each have a duration of 24 hours, are generated.

6. The method according to claim 4 or 5, **characterized in that** the number of chewing actions which take place in a time period are calculated by carrying out a Fourier transformation for this time period over the chronological profile of the measured accelerations, the fundamental frequency determined here is interpreted as being a chewing action frequency and is multiplied by the length of the time period.

7. The method according to claim 6, **characterized in that** acceleration is measured in three respective coordinate directions, and **in that** for a multiplicity of different directions which are assumed in the space and for the acceleration vectors which are measured at the individual measuring times the absolute value of that directional component of the respective acceleration vector is calculated, which is parallel to the respective direction assumed in the space, with the result that a data series is formed for each of the directions assumed in the space, over the chronological sequence of the acceleration components oriented in the respective direction, and **in that** the Fourier transformation is carried out at one or more of these data series.

8. The method according to claim 5 and claim 6 or claim 7, **characterized in that** the time period over which chewing actions are counted extends from the end of one swallowing phase to the start of the next swallowing phase.

9. The method according to any one of claims 1 to 8, **characterized in that** swallowing processes which are part of a drinking process are counted separately.

10. The method according to claim 9, **characterized in that** the process of drinking is identified by variance analysis of the measured acceleration data, wherein fluctuations in the variance profile within a predetermined frequency range are evaluated as an indication of a drinking process.

11. The method according to any one of claims 1 to 10, **characterized in that** said method is applied to a ruminant, and **in that** the acceleration data is also evaluated to the effect of detecting whether the swallowing processes and, if appropriate, chewing actions take place during the consumption of feed or during the rumination, and **in that** swallowing processes within the scope of the consumption of feed are counted separately from swallowing processes within the scope of ruminating.

12. The method according to claim 3 and any one of the further claims 1 to 11, **characterized in that** in an automated data processing operation one or more of the following information relating to the animal are generated on the basis of the measured counting values and further stored data:
- number of swallowing processes per day,
- number of swallowing processes during the consumption of feed per day,
- ratio of numbers of chewing actions per swallowing process during the rumination,
- ratio of numbers of chewing actions per swallowing process during the consumption of feed,
- number of swallowing processes while drinking,
- deviations from the average value of the individual previously mentioned numbers and ratios of numbers with respect to a standardized value and/or an average value for a herd,
- deviations from the average value of the previously mentioned numbers and ratios of numbers with respect to the average value for the same animal in preceding time periods,
- information about a state of the animal for which the previously mentioned numbers and/or ratios of numbers and/or deviations are an indication, in particular information about the time of mating, information about the predicted optimum insemination time period, information about the ratio of the long fiber content with respect to the protein content in the feed, information about the supposed milk yield, information about the state of health.

13. The method according to any one of claims 1 to 12, **characterized in that** the farm animal is a ruminant, and **in that** the recorded acceleration data is evaluated to the effect that the measured value of a quantifiable variable which describes an instantaneous state of a ruminating process is determined.

14. The method according to claim 13, **characterized in that** the quantified data which is obtained by evaluation relates to the frequency of the chewing actions during the rumination.

15. The method according to claim 13 or 14, **characterized in that** by evaluation of the acceleration data it is inferred whether the individual acceleration data are to be assigned to a chewing phase (K) or a swallowing/regurgitating phase (SW) of a ruminating process, and **in that** further evaluations are carried out only with that measurement data which is assigned to a chewing phase (K).

16. The method according to claim 15, **characterized in that** the variance of the measurement data or a variable which correlates to the variance of the measurement data is calculated over two time windows which adjoin one another and which each relate to a partial duration of the measuring duration of acceleration data, and **in that** a pronounced difference between the two variable values calculated therewith is interpreted as a change between a chewing phase (K) and a swallowing/regurgitating (SW) phase during the rumination.

17. The method according to claim 15 or 16, **characterized in that** the evaluation is used for the purpose of differentiating the assignment of the measurement data to a chewing phase (K) or a swallowing/regurgitating phase (SW) on the basis of the series of those chronologically successive acceleration values which each signify the absolute value of the total acceleration at a measuring time and can be calculated for the respective measuring time by geometric addition of the individual acceleration values measured in the individual coordination directions at the respective measuring time.

18. The method according to claim 7 and any one of claims 13 to 17, **characterized in that** each case a Fourier transformation is carried out for a multiplicity of data series, which are each assigned to another direction in the space, and **in that** from the obtained multiplicity of results that result is filtered out in which in each frequency range in which the frequency of the chewing actions can in principle occur, a single oscillation dominates to the greatest extent in terms of amplitude over all the other oscillations in this frequency range, and **in that** the frequency of this dominant oscillation is interpreted as the frequency of the chewing actions.

19. The method according to claim 7 and any one of claims 13 to 17, **characterized in that** each case a Fourier transformation is carried out for a multiplicity of data series, which are each assigned to another direction in the space, and **in that** from the obtained number of results that result is filtered out in which, in each frequency range in which the frequency of the chewing actions can in principle occur, three oscillations which occur one next to the other with respect to frequency dominate to the greatest extent in terms of amplitude over all the other oscillations in this frequency range, and **in that** that frequency at which, in the Fourier representation, the apex point of that parabola occurs with an axis of symmetry which is parallel to the ordinate, and which runs through those points which characterize said three oscillations with respect to the frequency and amplitude, is interpreted as the frequency of the chewing actions.

20. The method according to claim 15 and claim 18 or 19, **characterized in that** the number of chewing actions per portion of feed, which is regurgitated from the rumen into the mouth, chewed and then swallowed again is calculated by multiplying the frequency of the chewing actions by the duration of the chewing phase.

## Revendications

1. Procédé pour l'obtention d'informations sur un animal de rente, dans lequel est installé en la zone de tête d'un animal un appareil (a) qui renferme au moins un accéléromètre par lequel on mesure périodiquement des données d'accélération, dans lequel on évalue les données d'accélération avec des moyens de traitement de données automatisé et l'on tire des conclusions sur des activités et/ou des états de l'animal en tant que résultat de l'évaluation,
**caractérisé en ce que**
l'accéléromètre est disposé dans une marque auriculaire et qu'on évalue les données d'accélération enregistrées de manière à reconnaître des processus de déglutition effectués par l'animal.

2. Procédé selon la revendication 1, **caractérisé en ce qu'**on évalue des données d'accélération de manière à distinguer des périodes temporelles où l'animal déglutit de périodes temporelles où l'animal mastique, et qu'on calcule de plus les variances des données d'accélération mesurées dans des fenêtres temporelles contiguës les unes aux autres et on les compare.

3. Procédé selon la revendication 1 ou 2, **caractérisé en ce qu'**on compte les processus de déglutition.

4. Procédé selon l'une des revendications 1 à 3, **caractérisé en ce qu'**on évalue en plus les données d'accélération enregistrées de manière à reconnaître et compter des mastications effectuées par l'animal.

5. Procédé selon l'une des revendications 2 à 4, **caractérisé en ce qu'**on génère des valeurs numériques qui se rapportent chacune à une fenêtre temporelle prédéterminée, en particulier à des fenêtres temporelles d'une durée respective de 24 heures.

6. Procédé selon l'une des revendications 4 ou 5, **caractérisé en ce qu'**on calcule le nombre de mastications ayant lieu dans une fenêtre temporelle en effectuant pour cette fenêtre temporelle une transformation de Fourier sur l'évolution temporelle des accélérations mesurées, on interprète la fréquence de base ainsi établie comme fréquence de mastication et on la multiplie avec la longueur de la fenêtre temporelle.

7. Procédé selon la revendication 6, **caractérisé en ce qu'**on mesure à chaque fois l'accélération dans trois directions de coordonnées et qu'on calcule, pour une pluralité de directions prises dans l'espace différentes les unes des autres et pour les vecteurs d'accélération mesurés aux divers instants de mesure, la valeur de la composante directionnelle du vecteur d'accélération respectif qui est parallèle à la direction en question prise dans l'espace, si bien que pour chacune des directions prises dans l'espace, on forme à chaque fois une série de données sur la succession temporelle des composantes d'accélération orientées dans la direction en question, et qu'on effectue la transformation de Fourier sur une ou plusieurs desdites séries de données.

8. Procédé selon la revendication 5 et la revendication 6 ou la revendication 7, **caractérisé en ce que** la fenêtre temporelle pendant laquelle on compte des mastications va de la fin d'une phase de déglutition au début de la phase de déglutition suivante.

9. Procédé selon l'une des revendications 1 à 8, **caractérisé en ce qu'**on compte séparément des processus de déglutition qui font partie d'un processus d'abreuvement.

10. Procédé selon la revendication 9, **caractérisé en ce qu'**on identifie le processus d'abreuvement par une analyse de variance des données d'accélération mesurées, dans lequel on apprécie des fluctuations de l'évolution de variance dans une plage de fréquences prédéterminée en tant qu'indication sur un processus d'abreuvement.

11. Procédé selon l'une des revendications 1 à 10, **caractérisé en ce qu'**il s'applique à un ruminant et qu'on évalue les données d'accélération également de manière à reconnaître si les processus de déglutition et éventuellement des mastications ont lieu pendant la prise alimentaire ou pendant la rumination, et qu'on compte des processus de déglutition dans le cadre de la prise alimentaire séparément de processus de déglutition dans le cadre de la rumination.

12. Procédé selon la revendication 3 et l'une des autres revendications 1 à 11, **caractérisé en ce que** dans un processus de traitement de données automatisé, on génère une ou plusieurs des déclarations suivantes concernant l'animal sur la base des valeurs numériques mesurées et d'autres données sauvegardées :
- nombre des processus de déglutition par jour
- nombre des processus de déglutition lors de la prise alimentaire par jour
- rapport nombre de mastications par processus de déglutition lors de la rumination
- rapport nombre de mastications par processus de déglutition lors de la prise alimentaire
- nombre des opérations de déglutition lors de l'abreuvement
- écarts de la valeur moyenne des divers nombres ou rapports de nombres cités plus haut par rapport à une valeur standard et/ou une valeur moyenne de troupeau
- écarts de la valeur moyenne des nombres ou rapports de nombres cités plus haut par rapport à la valeur moyenne pour le même animal dans des fenêtres temporelles passées
- déclarations sur un état de l'animal pour lequel les nombres et/ou rapports de nombres et/ou écarts cités plus haut sont une indication, en particulier des déclarations sur la période de rut, des déclarations sur la période d'insémination optimale prévue, des déclarations sur le rapport entre la teneur en fibres longues et la teneur en protéines dans l'alimentation, des déclarations sur la production présumée de lait, des déclarations sur l'état de santé.

13. Procédé selon l'une des revendications 1 à 12, **caractérisé en ce que** l'animal de rente est un ruminant, et qu'on évalue les données d'accélération enregistrées de manière à relever la valeur de mesure d'une grandeur quantifiable qui décrit un état instantané d'un processus de rumination.

14. Procédé selon la revendication 13, **caractérisé en ce que** les données quantifiées obtenues par évaluation concernent la fréquence des mastications pendant la rumination.

15. Procédé selon la revendication 13 ou 14, **caractérisé en ce qu'**on conclut par l'évaluation des données d'accélération si les diverses données d'accélération sont à associer à une phase de mastication (K) ou une phase de déglutition-haut-le-cœur (5W) d'un processus de rumination et qu'on effectue d'autres évaluations seulement avec les données de mesure qui ont été associées à une phase de mastication (K).

16. Procédé selon la revendication 15, **caractérisé en ce qu'**on calcule la variance des données de mesure ou une grandeur en corrélation avec la variance des données de mesure sur deux fenêtres temporelles qui sont contiguës l'une à l'autre et concernent chacune une durée partielle de la durée de mesure de données d'accélération, et qu'on interprète une forte divergence des deux valeurs de grandeur ainsi calculées comme passage entre une phase de mastication (K) et une phase de déglutition-haut-le-cœur (SW) pendant la rumination.

17. Procédé selon la revendication 15 ou 16, **caractérisé en ce qu'**on utilise l'évaluation aux fins de distinguer l'association des données de mesure à une phase de mastication (K) ou une phase de déglutition-haut-le-cœur (SW) sur la base de la série des valeurs d'accélération se suivant les unes les autres dans le temps qui traduisent la valeur de l'accélération totale à un instant de mesure respectif et peuvent être calculées pour l'instant de mesure concerné par une addition géométrique des diverses valeurs d'accélération mesurées à l'instant de mesure concerné dans les diverses directions de coordonnées.

18. Procédé selon la revendication 7 ou l'une des revendications 13 à 17, **caractérisé en ce qu'**on effectue à chaque fois une transformation de Fourier pour une pluralité de séries de données qui sont associées chacune à une autre direction dans l'espace respective, et qu'on filtre parmi la pluralité de résultats obtenue celui pour lequel dans la plage de fréquences dans laquelle précisément la fréquence des mastications peut se situer en principe une oscillation unique en termes d'amplitude domine le plus fortement sur toutes les autres oscillations dans ladite plage de fréquences et qu'on interprète la fréquence de ladite oscillation dominante comme la fréquence des mastications.

19. Procédé selon la revendication 7 et l'une des revendications 13 à 17, **caractérisé en ce qu'**on effectue à chaque fois une transformation de Fourier pour une pluralité de séries de données qui sont associées chacune à une autre direction dans l'espace respective, et qu'on filtre parmi la pluralité de résultats obtenue celui pour lequel dans la plage de fréquences dans laquelle précisément la fréquence des mastications peut se situer en principe trois oscillations qui sont proches les unes des autres en termes de fréquence concernant l'amplitude dominent le plus fortement sur toutes les autres oscillations dans ladite plage de fréquences et qu'on interprète comme fréquence des mastications celle sur laquelle se situe, dans la représentation de Fourier, le point culminant de la parabole qui présente un axe de symétrie parallèle à l'ordonnée qui s'étend à travers les points qui caractérisent lesdites trois oscillations en termes de fréquence et d'amplitude.

20. Procédé selon la revendication 15 et la revendication 18 ou 19, **caractérisé en ce qu'**on calcule le nombre des mastications par portion alimentaire qui est régurgitée depuis la panse dans la gueule, mâchée puis à nouveau avalée en multipliant la fréquence des mastications par la durée de la phase de mastication.
